# FASCICULE DE BREVET EUROPEEN

(11) **EP 2 262 779 B1**
(45) Date de publication et mention de la délivrance du brevet: **05.12.2012**
(21) Numéro de dépôt: 09742271.1
(22) Date de dépôt: 08.04.2009
(51) Int. Cl.: C07D 233/34

(54) **PREPARATION DE (METH)ACRYLATES D'ALKYLIMIDAZOLIDONE DANS L'EAU**
HERSTELLUNG VON ALKYLIMIDAZOLIDON-(METH)ACRYLATEN IN WASSER
PREPARATION OF ALKYLIMIDAZOLIDONE (METH)ACRYLATES IN WATER

(30) Priorité: 17.04.2008 FR 0852594
(43) Date de publication de la demande: 22.12.2010
(73) Titulaire: ARKEMA FRANCE, 92700 Colombes (FR)
(72) Inventeur: RIONDEL, Alain, F-57600 Forbach (FR); PAUL, Jean-Michel, F-57070 Metz (FR)
(74) Mandataire: Bonnel, Claudine
(86) Numéro de dépôt international: PCT/FR2009/050615
(87) Numéro de publication internationale: WO 2009/136073

(56) Documents cités:
- EP-A- 0 902 017
- EP-A- 1 241 163
- US-A1- 2006 173 191

## Description

### Domaine de l'invention

L'invention concerne les monomères (méth)acryliques spéciaux et a trait plus particulièrement à un procédé amélioré de préparation de solutions de (méth)acrylates d'alkylimidazolidone dans l'eau.

### Technique antérieure

Les (méth)acrylates d'alkylimidazolidone sont connus pour leur rôle dans la constitution de polymères utilisables comme revêtements et adhésifs, dans le domaine du papier et des textiles, pour leur utilisation comme agents de traitements du cuir et dans la production de peintures en émulsion.

D'après les documents, par exemple EP 1 293 502, EP 433 135, EP 712 846, EP 650 962 ou EP 619 309, il est connu de préparer les acrylates ou les méthacrylates (désignés par (méth)acrylates) d'alkylimidazolidone de formule (I) : 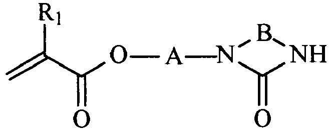 dans laquelle R₁ est un atome d'hydrogène ou un groupement méthyle, et A et B représentent, indépendamment l'un de l'autre, un groupement alkylène à chaîne droite ou ramifiée ayant de 2 à 5 atomes de carbone, selon un procédé de transestérification par réaction d'au moins un (méth)acrylate d'alkyle de formule (II) : 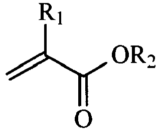 dans laquelle R₁ a la signification précitée et R₂ est un groupement alkyle à chaîne droite ou ramifiée ayant de 1 à 4 atomes de carbone, avec un alcool hétérocyclique de formule (III) : 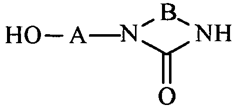 dans laquelle A et B ont les significations précitées, en présence d'un catalyseur et généralement en présence d'un inhibiteur de polymérisation.

Lorsque le (méth)acrylate (II) est en large excès par rapport à l'alcool hétérocyclique (III), on obtient à la fin de la réaction une composition comprenant une solution de (méth)acrylate d'alkylimidazolidone de formule (I) dans le (méth)acrylate (II).

Ainsi, les (méth)acrylates d'alkylimidazolidone sont généralement commercialisés dans un (méth)acrylate léger (II). Plus particulièrement, le méthacrylate d'éthyl-1-imidazolyl-2-one (MEIO), préparé à partir de méthacrylate de méthyle (MAM), est commercialisé en solution dans le méthacrylate de méthyle (MAM).

Les (méth)acrylates d'alkylimidazolidone en solution dans un (méth)acrylate léger, tel que le méthacrylate de méthyle, présentent cependant des inconvénients lors de leur mise en oeuvre. En particulier, selon l'utilisation finale, le méthacrylate de méthyle possède une faible tension de vapeur et conduit à l'émission de composés organiques volatiles (VOC).

Il est connu de substituer le méthacrylate de méthyle par de l'eau dans des compositions comprenant un (méth)acrylate d'alkylimidazolidone et du méthacrylate de méthyle, obtenues selon un procédé de transestérification en présence d'un inhibiteur de polymérisation.

Selon le document EP 1 241 163, des solutions de méthacrylate d'éthyl-1-imidazolidyl-2-one dans du MAM sont préparées par transestérification en présence de 4-hydroxy-2,2,6,6-tétraméthyl piperidinyloxy (4-hydroxy TEMPO) comme inhibiteur de polymérisation. Puis la méthode de substitution du MAM par l'eau consiste à éliminer la quasi-totalité du méthacrylate de méthyle par distillation sous vide dans des conditions de température/pression de 40°C à 60°C / 760 à 60 mm de Hg (1013 à 80 mbar), puis, après avoir refroidi le produit à une température de 60°C-70°C, à ajouter de l'eau en maintenant le mélange sous agitation pendant 15 minutes. Il n'est pas ajouté d'inhibiteur de polymérisation pour cette étape. La composition finale obtenue contient plus de 50 % d'eau mais encore plus de 2% de méthacrylate de méthyle résiduel.

Selon le document EP 902 017, la méthode consiste à éliminer le méthacrylate de méthyle par distillation sous forme d'azéotrope avec l'eau, alors que l'introduction de l'eau dans le mélange comprenant le (méth)acrylate d'alkylimidazolidone et le méthacrylate de méthyle se fait en continu. La composition finale obtenue contient environ 48 % d'eau et moins de 1% de méthacrylate de méthyle résiduel. Cependant, ce procédé entraîne la distillation de grandes quantités d'azéotrope MAM / eau, et nécessite une décantation, puis une filtration sous vide du produit final pour obtenir un produit limpide.

Il a maintenant été trouvé un procédé amélioré pour substituer le (méth)acrylate léger par de l'eau dans les formulations de (méth)acrylates d'alkylimidazolidone dans un (méth)acrylate léger. L'amélioration du procédé consiste à éliminer une quantité suffisante de (méth)acrylate léger par distillation avant d'introduire de l'eau en continu et poursuivre l'élimination du (méth)acrylate léger sous forme d'azéotrope (méth)acrylate léger / eau. En outre, la distillation du (méth)acrylate léger est réalisée après ajout d'une quantité efficace d'au moins un inhibiteur de polymérisation à l'exception des dérivés du 2,2,6,6-tétraméthyl-1-piperidinyloxy (TEMPO). En effet, la présence d'un dérivé du TEMPO peut affecter la coloration du monomère, et elle est préjudiciable pour l'utilisation de la solution finale de (méth)acrylate d'alkylimidazolidone dans l'eau, notamment pour sa réactivité pour préparer des polymères à base de (méth)acrylate d'alkylimidazolidone.

Cette façon de procéder permet ainsi de pallier les inconvénients des procédés existants précités tout en fabriquant un produit stable thermiquement sans mettre en oeuvre de composé néfaste pour l'utilisation finale du produit obtenu.

La présente invention a donc pour but de fournir un procédé de préparation de solutions de (méth)acrylates d'alkylimidazolidone dans l'eau, qui soit simple et rapide (comportant le moins d'étapes possibles, telles que décantation ou filtration), avec des besoins énergétiques réduits et conduisant à un produit de haute qualité avec une productivité améliorée.

### Description de l'invention

La présente invention a donc pour objet un procédé de préparation de solutions dans l'eau de (méth)acrylate d'alkylimidazolidone de formule (I) 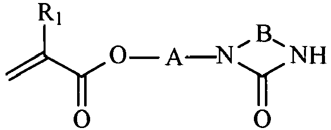 dans laquelle R₁ est un atome d'hydrogène ou un groupement méthyle, et A et B représentent, indépendamment l'un de l'autre, un groupement alkylène à chaîne droite ou ramifiée ayant de 2 à 5 atomes de carbone,
à partir de solutions de (méth)acrylate d'alkylimidazolidone de formule (I) dans un (méth)acrylate léger de formule (II) 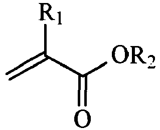 dans laquelle R₁ est un atome d'hydrogène ou un groupement méthyle et R₂ est un groupement alkyle à chaîne droite ou ramifiée ayant de 1 à 4 atomes de carbone, caractérisé en ce qu'il comprend les étapes suivantes :
(a) on ajoute dans la solution initiale de 100 à 2000 ppm d'au moins un inhibiteur de polymérisation, à l'exception des dérivés du 2,2,6,6-tétraméthyl-1-piperidinyloxy.
(b) on élimine par distillation sous pression réduite de 80 à 90 en poids du (méth)acrylate de formule (II) présent dans la solution initiale.
(c) on ajoute en continu de l'eau en même temps que l'on poursuit la distillation de façon à éliminer le (méth)acrylate de formule (II) restant sous forme d'azéotrope (méth)acrylate de formule (II) / eau.
(d) on ramène le mélange obtenu en (c) à pression atmosphérique et à température ambiante et on introduit une quantité d'eau pour ajuster le titre en eau de la solution finale.

La solution initiale de (méth)acrylate d'alkylimidazolidone de formule (I) dans le (méth)acrylate léger (II) contient en général de 20 à 80 % en poids exprimés en (méth)acrylate d'alkylimidazolidone de formule (I) avec des sous-produits monomériques porteurs d'une fonction uréido, par rapport à la solution initiale, de préférence, de 30 à 60 % en poids, et plus particulièrement de 45 à 55 % en poids. Les sous-produits monomériques porteurs d'une fonction uréido sont les sous-produits inhérents à la fabrication du (méth)acrylate d'alkylimidazolidone de formule (I) par transestérification. Ils résultent de l'addition de type Michaël de la fonction amine secondaire du cycle imidazolidone sur une autre molécule de (méth)acrylate d'alkylimidazolidone (I) ou sur une molécule de (méth)acrylate d'alkyle (II). Généralement, ces sous-produits sont présents à une teneur allant de 5 à 25 % par rapport au (méth)acrylate d'alkylimidazolidone, en particulier de 10 à 20 %.

Comme exemples de composé (I), on peut citer ceux pour lesquels le groupement A est un groupement alkylène ayant 2 atomes de carbone, et plus particulièrement le méthacrylate d'éthyl-1- imidazolidyl-2-one (MEIO).

De préférence, le (méth)acrylate (II) est un (méth)acrylate léger tel que le (méth)acrylate de méthyle ou le (méth)acrylate d'éthyle, présent à une teneur allant de 20 à 80% dans la solution initiale, particulièrement de 40 à 70%, plus précisément de 15 à 55% en poids dans la solution initiale.

Les solutions initiales de (méth)acrylate d'alkylimidazolidone de formule (I) dans un (méth)acrylate léger de formule (II) peuvent être obtenues selon l'un des procédés connus de l'homme du métier, tels que ceux cités précédemment, ou sont disponibles commercialement.

Le procédé selon l'invention consiste à substituer le (méth)acrylate léger de formule (II) par de l'eau, en quatre étapes.

La première étape (a) consiste à charger la solution initiale de (méth)acrylate d'alkylimidazolidone dans le (méth)acrylate léger dans un réacteur et à ajouter un ou plusieurs inhibiteurs de polymérisation en dehors des dérivés du 2,2,6,6-tétraméthyl-1-piperidinyloxy (TEMPO) On peut ajouter par exemple de la phénothiazine, de l'hydroquinone, de l'éther monométhylique d'hydroquinone, du diterbutyl para-crésol, de la paraphénylène diamine, ou du di-tertiobutylcatéchol. La teneur en inhibiteurs de polymérisation est généralement comprise entre 100 et 2000 ppm dans la solution, de préférence de 200 à 800 ppm.

La seconde étape (b) consiste à distiller de 80 à 90 % du (méth)acrylate léger présent dans la solution de (méth)acrylate d'alkylimidazolidone . Généralement, la distillation est réalisée à une température allant de 35°C à 65°C, de préférence de 40°C à 55°C, sous une pression comprise entre 50 et 80 mm Hg (67 et 107 mbar), pendant 1 à 5 heures.

Puis, la troisième étape (c) consiste à introduire de l'eau en continu, tout en continuant la distillation du (méth)acrylate léger. Généralement, on ajoute la quantité d'eau nécessaire pour distiller sous forme d'azéotrope le (méth)acrylate léger restant, soit une quantité d'eau allant de 10 % à 50 %, de préférence de 15% à 40% exprimés par rapport au (méth)acrylate léger initial. L'eau est de préférence additivée d'au moins un inhibiteur de polymérisation, généralement choisi parmi ceux déjà ajoutés dans la solution initiale. L'addition est faite en continu sur une durée pouvant aller de 0,5 à 3 heures, sous une pression de 60 à 80 mm Hg (80 à 107 mbar) et à une température allant de 35°C à 50°C.

Durant cette troisième étape, l'azéotrope (méth)acrylate léger / eau est éliminé et la teneur résiduelle en (méth)acrylate (II) dans la solution obtenue est généralement inférieure à 0,5 %, voire inférieure à 0,2%. Il n'est donc pas nécessaire d'effectuer un strippage final, pour compléter l'élimination du composé léger. Cette étape, du fait de la quantité limitée de (méth)acrylate léger à éliminer ne nécessite pas la mise en oeuvre de grandes quantités d'eau et par conséquent ne dure pas longtemps.

Pendant ces deux étapes (b) et (c), avantageusement un bullage d'air, éventuellement appauvri (8 à 9 % O₂ en volume) est effectué.

A l'issue de la troisième étape, au cours de l'étape (d), la solution est ramenée à la pression atmosphérique et à température ambiante, et une quantité d'eau est ajoutée de façon à obtenir une solution finale comprenant de 40 à 60% d'eau, de préférence de 45 à 55% d'eau. La teneur en eau peut être déterminée facilement à l'aide d'une thermobalance.

Les solutions dans l'eau ainsi obtenues restent stables sur une longue durée et peuvent être utilisées pour préparer des polymères (méth)acryliques spéciaux.

Les exemples suivants illustrent la présente invention sans toutefois en limiter la portée.

### Exemples de réalisation

Les abréviations suivantes y sont utilisées :
HEIO : 1-(2-hydroxyéthyl)-imidazolidyl-2-one
MEIO : méthacrylate d'éthyl-1- imidazolidyl-2-one
NORSOCRYL^{©}104 : solution à 50 % massique de méthacrylate d'éthyl-1-imidazolidyl-2-one et d'impuretés monomériques du type uréido dans du méthacrylate de méthyle.
MAM : méthacrylate de méthyle
EMHQ : éther méthylique d'hydroquinone
PTZ : phénothiazine

Les pourcentages sont exprimés en pourcentages massiques.

### Exemple 1 (selon l'invention)

On utilise comme appareillage un réacteur en verre de 1L double enveloppe équipé d'une colonne adiabatique à garnissage multiknit (environ 12 plateaux) surmonté d'une tête de reflux, de deux sondes pour la mesure de la température en tête de colonne et dans le réacteur, d'une agitation mécanique à vitesse variable, d'une ampoule de coulée. Le chauffage est assuré par un bain d'huile.

On charge dans le réacteur 1114 g de Norsocryl^{©} 104 (N104) et 0,409 g de EMHQ (367 ppm par rapport au N104). La composition du N104 est la suivante :
- HEIO : 0,7%
- MEIO : 40,8%
- MAM : 50,2%
- EMHQ : 92 ppm
- PTZ : 411 ppm
- Autres produits : 8,3%

On maintient un bullage d'air au sein de la charge et on met en oeuvre les conditions opératoires suivantes pour distiller le MAM contenu dans le N104 :
P : 75 mm Hg (100 mbar)
Température du réacteur : 41 à 57°C (température limite haute fixée à 65°C)
Température tête de colonne : 35°C maxi
Taux de reflux : 1/1
Durée : 1h30
On distille 476,6 g de MAM, ce qui équivaut à 85,2% du MAM présent.

Lorsque le débit en tête de colonne est pratiquement nul, on commence l'introduction de l'eau, soit 210,4 g d'eau additivée de 0,322 g de EMHQ (soit 1530 ppm) ajoutés sur une durée de 30 minutes, dans les conditions suivantes :
P : 75 mm Hg (100 mbar)
Température du réacteur : 40 à 46°C
Température tête de colonne : 28-40°C
Taux de reflux : 10/1

Au cours de cette étape qui dure 1 heure, l'azéotrope MAM/eau est distillé, et lorsque la température en tête de colonne s'aligne sur celle de l'eau, on distille 40 g d'eau pour bien épuiser le MAM en pied pendant une heure et avec un taux de reflux de 10/1.

Le distillat décante en deux phases, on récupère 55 g de MAM dans la phase supérieure et 60 g de phase aqueuse inférieure.

Après mise hors service de la chauffe, le réacteur est remis à pression atmosphérique. Et après retour à la température ambiante, on ajoute 160,7 g d'eau et on contrôle la teneur en eau par thermobalance. Puis on complète l'addition d'eau pour obtenir un titre en eau de l'ordre de 48%. On a ajouté 55,7 g d'eau ce qui a conduit à un titre final en eau de 48,3%.

On récupère 960 g d'une solution dont l'analyse HPLC indique la composition suivante :
- HEIO : 1,3%
- MEIO : 46,6%
- MAM : 0,2%
- EMHQ : 754 ppm
- PTZ : 409 ppm
- eau : 48,3%
- Autres produits : 3,7%

La solution obtenue est stable et on n'observe pas de polymérisation après 12 jours de maintien à une température de 60°C.

### Exemple 2 (comparatif)

On reproduit les conditions opératoires de l'exemple 1, sauf que, toutes choses étant égales par ailleurs, on élimine 532 g du MAM (soit 95%) présent dans le N104 de départ, sur une durée de 1h45. Dans ces conditions, la stabilité de la solution aqueuse obtenue est inférieure à 24h.

### Exemple 3 (comparatif)

On reproduit les conditions opératoires de l'exemple 1, sauf que, toutes choses étant égales par ailleurs, on élimine 391,5 g du MAM (soit 70%) présent dans le N104 de départ, sur une durée d'une heure. Dans ces conditions, la durée d'élimination de l'azéotrope MAM/eau est de 4h30, ce qui entraine une diminution de la productivité de l'ordre de 40%.

## Revendications

1. Procédé de préparation de solutions dans l'eau de (méth)acrylate d'alkylimidazolidone de formule (I) 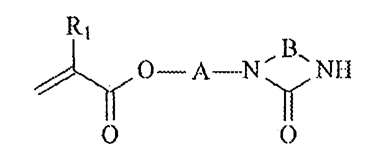 dans laquelle R₁ est un atome d'hydrogène ou un groupement méthyle, et A et B représentent, indépendamment l'un de l'autre, un groupement alkylène à chaîne droite ou ramifiée ayant de 2 à 5 atomes de carbone,
à partir de solutions de (méth)acrylate d'alkylimidazolidone de formule (I) dans un (méth)acrylate léger de formule (II) 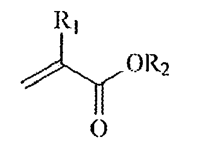 dans laquelle R₁ est un atome d'hydrogène ou un groupement méthyle et R₂ est un groupement alkyle à chaîne droite ou ramifiée ayant de 1 à 4 atomes de carbone, **caractérisé en ce qu'**il comprend les étapes suivantes :
(a) on ajoute dans la solution initiale de 100 à 2000 ppm d'au moins un inhibiteur de polymérisation, à l'exception des dérivés du 2,2,6,6-tétraméthyl-1-piperidinyloxy.
(b) on élimine par distillation sous pression réduite de 80 à 90% en poids du (méth)acrylate de formule (II) présent dans la solution initiale.
(c) on ajoute en continu de l'eau en même temps que l'on poursuit la distillation de façon à éliminer le (méth)acrylate de formule (II) restant sous forme d'azéotrope (méth)acrylate de formule (II) / eau.
(d) on ramène le mélange obtenu en (c) à pression atmosphérique et à température ambiante et on introduit une quantité d'eau pour ajuster le titre en eau de la solution finale.

2. Procédé selon la revendication 1 **caractérisé en ce que** le composé de formule (I) est le méthacrylate d'éthyl-1- imidazolidyl-2-one.

3. Procédé selon la revendication 1 ou 2 **caractérisé en ce que** la quantité d'eau ajoutée en continu à l'étape (c) va de 10 % à 50 % exprimés par rapport au (méth)acrylate léger initial.

4. Procédé selon l'une quelconque des revendications précédentes **caractérisé en ce que** le titre en eau de la solution finale est ajusté de 40% à 60%.

## Claims

1. A process for preparing solutions, in water, of an alkylimidazolidone (meth)acrylate of formula (I) : 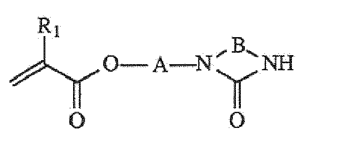 in which R₁ is a hydrogen atom or a methyl group and A and B represent, independently of one another, a straight- or branched-chain alkylene group having from 2 to 5 carbon atoms,
from solutions of an alkylimidazolidone (meth)-acrylate of formula (I) in a light (meth)acrylate of formula (II) : 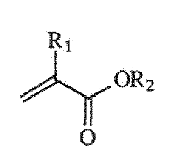 in which R₁ is a hydrogen atom or a methyl group and R₂ is a straight- or branched-chain alkyl group having from 1 to 4 carbon atoms, **characterized in that** it comprises the following steps:
(a) adding, to the initial solution, from 100 to 2000 ppm of at least one polymerization inhibitor, with the exception of 2,2,6,6-tetramethyl-1-piperidinyloxy derivatives;
(b) removing, by distillation under reduced pressure, from 80 to 90% by weight of the (meth)acrylate of formula (II) present in the initial solution;
(c) continuously adding water at the same time as the distillation is continued in order to remove the remaining (meth)acrylate of formula (II) in the form of a (meth) - acrylate of formula (II)/water azeotrope; and
(d) bringing the mixture obtained in (c) back to atmospheric pressure and to ambient temperature and introducing an amount of water in order to adjust the water content of the final solution.

2. The process as claimed in claim 1, **characterized in that** the compound of formula (I) is 1-ethyl-imidazolidyl-2-one methacrylate.

3. The process as claimed in claim 1 or 2, **characterized in that** the amount of water added contiguously in step (c) ranges from 10% to 50% expressed relative to the initial light (meth) - acrylate.

4. The process as claimed in any one of the preceding claims, **characterized in that** the water content of the final solution is adjusted to 40% to 60%.

## Patentansprüche

1. Verfahren zur Herstellung von Lösungen von Alkyl-imidazolidon(meth)acrylaten der Formel (I) 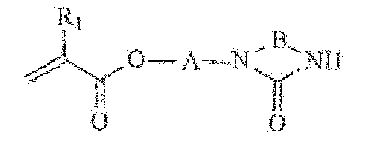 worin R₁ für ein Wasserstoffatom oder eine Methylgruppe steht und A und B unabhängig voneinander für eine gerad- oder verzweigtkettige Alkylengruppe mit 2 bis 5 Kohlenstoffatomen stehen,
in Wasser ausgehend von Lösungen von Alkylimidazolidon (meth)acrylaten der Formel (I) in einem leichten (Meth)acrylat der Formel (II) 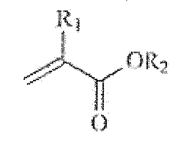 worin R₁ für ein Wasserstoffatom oder eine Methylgruppe steht und R₂ für eine gerad- oder verzweigtkettige Alkylgruppe mit 1 bis 4 Kohlenstoffatomen steht,
**dadurch gekennzeichnet, dass** man
(a) die Ausgangslösung mit 100 bis 2000 ppm mindstens eines Polymerisationsinhibitors mit Aufnahme von 2,2,6,6-Tetramethyl-1-piperidinyloxy-Derivaten versetzt,
(b) 80 bis 90 Gew.-% des in der Ausgangslösung vorliegenden (Meth)acrylats der Formel (II) unter vermindertem Druck abdestilliert;
(c) kontinuierlich Wasser zugibt und gleichzeitig die Destillation zur Entfernung des restlichen (Meth)acrylats der Formel (II) als Azeotrop aus dem (Meth)acrylat der Formel (II) und Wasser weiterführt,
(d) das in (c) erhaltene Gemisch wieder auf Atmosphärendruck und Umgebungstemperatur bringt und eine Menge Wasser zur Einstellung des Wassergehalts der fertigen Lösung zugibt.

2. Verfahren nach Anspruch 1, **dadurch gekennzeichnet, dass** es sich bei der Verbindung der Formel (I) um Ethyl-1-imidazolidyl-2-onmethacrylat handelt.

3. Verfahren nach Anspruch 1 oder 2, **dadurch gekennzeichnet, dass** die in Schritt (c) kontinuierlich zugegebene Wassermenge 10% bis 50%, bezogen auf das anfänglich vorliegende leichte (Meth)acrylat, beträgt.

4. Verfahren nach einem der vorhergehenden Ansprüche, **dadurch gekennzeichnet, dass** man den Wassergehalt der fertigen Lösung auf 40% bis 60% einstellt.
